# EUROPÄISCHE PATENTANMELDUNG

(11) **EP 1 563 796 A2**
(43) Veröffentlichungstag der Anmeldung: **17.08.2005**
(21) Anmeldenummer: 05002058.5
(22) Anmeldetag: 01.02.2005
(51) Int. Cl.: A61B 17/32, A61B 17/16, A61B 17/56

(54) **Satz von Operationsinstrumenten für Wirbelsäulenoperationen**

(30) Priorität: 10.02.2004 DE 102004006521
(71) Anmelder: Dekkers, Horst, Dr., 81737 München (DE)
(72) Erfinder:
(74) Vertreter: Jordan, Volker, Dr.

(57) **Zusammenfassung**

Die Erfindung betrifft ein Satz von Operationsinstrumenten zur Verwendung bei Wirbelsäulenoperationen, insbesondere Halswirbelsäulenoperationen, der eine Arbeitskanüle (2) und wenigstens ein Fräsinstrument (36; 136; 236) umfasst. Das Fräsinstrument (36; 136; 236) ist derart in ein Einführende (4) der Arbeitskanüle (2) einführbar, dass ein Fräsende (44; 144; 244) aus einem Austrittsende (6) der Arbeitskanüle (2) vorsteht und dass an einem Betätigungsende (46; 146; 246) des Fräsinstruments (36; 136; 236) Betätigungskräfte auf das Fräsinstrument (36; 136; 236) ausübbar sind.

## Beschreibung

Die vorliegende Erfindung bezieht sich auf ein Satz von Operationsinstrumenten für Wirbelsäulenoperationen, vorzugsweise der Halswirbelsäule, beispielsweise für Operationen im Zusammenhang mit Bandscheibenvorfällen.

Ein Satz von Operationsinstrumenten ist aus der WO00/76409 A1 bekannt. Dieser bekannte Satz von Operationsinstrumenten weist einen Satz von Trokarstäben mit fortschreitend erhöhtem Außendurchmesser auf und einen entsprechenden Satz von Hohlsägeinstrumenten, wobei jedes dieser Hohlsägeinstrumente mit seinem Innenumfang passgenau über den Außendurchmesser seines entsprechenden Trokarstabs schiebbar ist und auf dem Stab drehbar ist, um sich durch Knochenmaterial hindurch zu bohren. Die Hohlsägeinstrumente sind beim Zuführen zu einem Operationsbereich über den jeweiligen Trokarstab und beim eigentlichen Sägen im Wesentlichen auf ihrer ganzen Länge und mit ihrem gesamten Umfang mit umgebendem Körpergewebe in Berührung. Ferner umfasst dieser Satz von Operationsinstrumenten eine Arbeitskanüle, die nach dem Ausfräsen eines entsprechenden Operationskanals und nach Entfernung des Hohlsägeinstruments über die Trokarstange hin zum Bereich des zu behebenden Bandscheibenvorfalls geführt wird. Die Arbeitskanüle weist einen Außendurchmesser auf, der dem Außendurchmesser des größten Hohlsägeinstruments entspricht.

Aufgabe der Erfindung ist es, ein Satz von Operationsinstrumenten zur Verfügung zu stellen, der sich durch einfache Handhabbarkeit für den Operateur und durch Schonung des Patienten auszeichnet.

Zur Lösung dieser Aufgabe wird ein Satz von Operationsinstrumenten für Wirbelsäulenoperationen, vorzugsweise der Halswirbelsäule, bereitgestellt, der umfasst: eine Arbeitskanüle, die ein Einführende und Austrittsende und einen das Einführende und das Austrittsende verbindenden inneren Zuführungsbereich aufweist, wenigstens ein Fräsinstrument mit einem Fräsende und einem Betätigungsende, wobei das Fräsinstrument durch das Einführende derart in den Zuführungsbereich einführbar ist, dass das Fräsende aus dem Austrittsende vorsteht und dass an dem aus dem Einführende vorstehenden Betätigungsende im Sinne einer Drehbewegung relativ zur Arbeitskanüle oder/und im Sinne einer Verschiebebewegung relativ zur Arbeitskanüle wirkende Betätigungskräfte auf das Fräsinstrument ausübbar sind.

Ein derartiger Satz von Operationsinstrumenten ermöglicht ein vereinfachtes Wegfräsen von Knochenmaterial oder/und von durch einen Bandscheibenvorfall ausgetretenem Material des inneren Teils einer Bandscheibe (nucleus pulposus). Ferner kann durch die Fräse ein Zugangskanal zu einem Vorfall, der wenigstens teilweise durch einen Knochen der Wirbelsäule verdeckt ist, gefräst werden.

Da erfindungsgemäß das Fräsende des in die Arbeitskanüle eingeführten Fräsinstruments aus dem Austrittsende der Arbeitskanüle vorsteht, wird das Wegfräsen von Knochenmaterial oder/und Material eines Bandscheibenvorfalls gezielt in einem Operations-/Vorfallsbereich, der sich dem Austrittsende der Arbeitskanüle anschließt, ermöglicht. Dadurch wird gewährleistet, dass nur eine minimal notwendige Menge von Knochenmaterial bzw. Material eines Bandscheibenvorfalls entfernt werden kann, und es kann auf Grundlage einer vorzugsweise vorgesehenen Anschlagfunktion die Gefahr von unbeabsichtigten Verletzungen vermieden werden. Durch die Einführung des Fräsinstruments in die Arbeitskanüle kann die Verwendung eines dem Fräsinstrument angepassten Trokarrohres, wie dies aus dem oben erwähnten Stand der Technik bekannt ist, entfallen. Die am vorstehenden Betätigungsende ausgeübten Betätigungskräfte können unter der Führung des Fräsinstruments durch die Arbeitskanüle gezielt und kontrolliert das Fräsende betätigen, so dass einerseits eine einfache Handhabung für den Operateur und andererseits ein für den Patienten schonender minimal operativer Eingriff ermöglicht wird.

Das Fräsinstrument kann einen Außenumfang aufweisen, der an einem Innenumfang der Arbeitskanüle linear führbar ist. Hierdurch lässt sich eine im Wesentlichen spielfreie, nämlich radialspielfreie und kippspielfreie Bewegung des Fräsinstruments definieren, die dazu führt, dass ein der vorgegebenen Richtung der Bewegung entsprechender Operationsbereich gezielt bearbeitet werden kann.

Es ist bevorzugt, dass der Satz mehrere Fräsinstrumente unterschiedlicher Längen umfasst. Bei Verwendung von Fräsinstrumenten mit zunehmender Länge nacheinander ermöglicht dies die sukzessive Verlängerung des durch die Fräsinstrumente weggefrästen Operationskanals und im Weiteren den Zugang zu im Bezug auf das Austrittsende tiefer liegenden Operations-/Vorfallsbereichen, was besonders schonend ist.

Insbesondere stellt die Erfindung somit bereit ein Satz von Operationsinstrumenten für Wirbelsäulenoperationen, umfassend eine Arbeitskanüle, die ein Einführende und Austrittsende und einen das Einführende und das Austrittsende verbindenden inneren Zuführungsbereich aufweist, mehrere Fräsinstrumente unterschiedlicher Länge, jeweils mit einem Fräsende und einem Betätigungsende, wobei die Fräsinstrumente jeweils einen Außenumfang aufweisen, der an einem Innenumfang der Arbeitskanüle linear führbar ist, wobei die Fräsinstrumente jeweils durch das Einführende derart in den Zuführungsbereich einführbar sind, dass das jeweilige Fräsende aus dem Austrittsende vorsteht, wobei die Fräsinstrumente unterschiedlicher Länge bei maximaler Einführung in die Arbeitskanüle mit ihrem Fräsende unterschiedlich weit aus dem Austrittsende vorstehen, und dass an dem jeweiligen aus dem Einführende vorstehenden Betätigungsende im Sinne einer Drehbewegung relativ zur Arbeitskanüle oder/und im Sinne einer Verschiebebewegung relativ zur Arbeitskanüle wirkende Betätigungskräfte auf das jeweilige Fräsinstrument ausübbar sind.

In diesem Zusammenhang weisen die Fräsinstrumente unterschiedlicher Länge vorteilhafterweise das jeweilige Fräsinstrument identifizierende, optisch oder/und durch Tasten wahrnehmbare Kennzeichnungen auf. Die Kennzeichnungen ermöglichen die rasche Identifikation eines entsprechenden Fräsinstruments, wobei die Identifikation in der Regel einerseits durch den Operateur und andererseits durch dem Operateur beistehendes Assistenzpersonal erfolgt. Ferner wird durch eine solche Kennzeichnung die Handhabung des Satzes von Operationsinstrumenten vereinfacht.

Man kann vorteilhaft vorsehen, dass - wie schon angedeutet - das Fräsinstrument oder wenigstens eines der Fräsinstrumente am Betätigungsende oder zu diesem benachbart einen bezogen auf die Längserstreckung des Fräsinstruments ortsfesten Anschlag aufweist, der mit einem Anschlag der Arbeitskanüle an deren Einführende oder zu diesem benachbart zusammenwirkt, um eine maximale Länge des Vorstehens des Fräsendes aus dem Austrittsende zu definieren. Durch den Einsatz des ortsfesten Anschlags kann genau definiert werden, wie weit das jeweilige Fräsinstrument aus der Arbeitskanüle vorstehen kann, wodurch eine hohe Sicherheit erreicht wird. Im Falle der Fräsinstrumente mit unterschiedlichen Längen kann mit zunehmender Länge der Fräsinstrumente auch eine zunehmende maximale Länge des Vorstehens des Fräsendes aus dem Austrittsende definiert werden, die im Hinblick auf einen typischen Bandscheibenvorfall gerade so bemessen ist, dass die Fräse tief in den Vorfall hineinreichen kann. Diese Bemessung kann beispielsweise davon ausgehen, dass die Arbeitskanüle knapp vor dem Vorfall endet. Ferner kann es auch angebracht sein, eine längere Fräse einzusetzen, die deutlich weiter aus der Arbeitskanüle vorstehen kann und insbesondere die gesamte Breite einer Bandscheibe überbrücken kann. Dies ermöglicht eine Behandlung eines Bandscheibenvorfalls, auch wenn die Arbeitskanüle nicht bis knapp vor den Vorfall eingeführt werden kann.

Das Fräsinstrument oder wenigstens eines der Fräsinstrumente kann ferner am Betätigungsende oder zu diesem benachbart einen bezogen auf die Längserstreckung des Fräsinstruments verstellbaren, wahlweise fixierbaren Anschlag aufweisen, der mit einem Anschlag der Arbeitskanüle an deren Einführende oder zu diesem benachbart zusammenwirkt, um eine gewählte maximale Länge des Vorstehens des Fräsendes aus dem Austrittsende zu definieren. Durch den verstellbaren, wahlweise fixierbaren Anschlag kann eine maximale Eindringtiefe des Fräsinstruments bestimmt werden. Dies ermöglicht beispielsweise speziell bei Verwendung des angesprochenen längeren Fräsinstruments, dessen maximale Länge des Vorstehens allerdings ebenfalls durch einen ortsfesten Anschlag definiert sein kann, eine kleinere bzw. auf die Operationssituation angepasste Länge des Vorstehens des Fräsendes vermittels des verstellbaren, wahlweise fixierbaren Anschlags festzulegen, um den Operations-Norfallsbereich nicht unnötig zu vergrößern bzw. um nicht zu tief in den Operationsbereich vorzustoßen.

Es ist bevorzugt, dass der verstellbare, wahlweise fixierbare Anschlag auf dem Außenumfang des Fräsinstruments angeordnet und an diesem vorzugsweise mittels wenigstens einer Rändelschraube festklemmbar ist. Die Anordnung des verstellbaren Anschlags auf dem Außenumfang des Fräsinstruments in Verbindung mit der zum Festklemmen vorgesehenen Rändelschraube führt zu einer einfachen Handhabung des Fräsinstruments, insbesondere des längeren Fräsinstruments.

Zweckmäßig kann das Fräsinstrument oder wenigstens eines der Fräsinstrumente als Hohlinstrument mit einem ringsägeartigen Fräsabschnitt am Fräsende ausgeführt sein. Diese Ausführung ermöglicht durch den ringsägeartigen Fräsabschnitt das Wegfräsen von Knochenmaterial oder/und Material des Bandscheibenvorfalls auf Grundlage von am Betätigungsende im Sinne einer Drehbewegung relativ zur Arbeitskanüle oder/und im Sinne einer Verschiebebewegung relativ zur Arbeitskanüle wirkenden Betätigungskräften. Es ist dabei von Vorteil, dass das Hohlinstrument beim Drehen und Verschieben weggefrästes Material wenigstens teilweise im Hohlraum aufnehmen kann. Dadurch kann ein Teil des weggefrästen Materials bereits beim Entfernen des Fräsinstruments aus dem Operations-Norfallsbereich entnommen werden.

Es ist besonders vorteilhaft, wenn das Fräsinstrument oder wenigstens eines der Fräsinstrumente ein knauf- oder knebelartiges Betätigungsende aufweist. Mit dieser Ausbildung können die auf das Fräsinstrument wirkenden Betätigungskräfte durch entsprechende Bewegungen der Hand des Operateurs effektiv übertragen werden. Im Weiteren wird auf diese Art und Weise das Drehen der Fräsinstrumente erleichtert, was zu einer einfachen Handhabung des Fräsinstruments führt.

Es ist bevorzugt, dass der Satz ferner wenigstens ein Arbeitsinstrument mit einem löffelartigen oder/und schneidenartigen oder/und schaberartigen Arbeitsabschnitt umfasst, welches durch das Einführende derart in den Zuführungsbereich einführbar ist, dass der Arbeitsabschnitt aus dem Austrittsende vorsteht und dass an einem aus dem Einführende vorstehenden Betätigungsende im Sinne einer Drehbewegung relativ zur Arbeitskanüle oder/und im Sinne einer Verschiebebewegung relativ zur Arbeitskanüle wirkende Betätigungskräfte auf das Instrument ausübbar sind. Das Arbeitsinstrument dient in der Regel dazu, aus dem gefrästen Kanal darin enthaltenes Knochenmaterial bzw. Material des Bandscheibenvorfalls wegzunehmen bzw. den Operationskanal zu vergrößern. Ferner kann mit einem solchen Arbeitsinstrument nicht nur der Operationskanal insgesamt bearbeitet werden, wie dies durch das Wegfräsen mit dem Fräsinstrument üblich ist, sondern es kann auch nur ein Teilbereich des Operationskanals bzw. des Vorfallbereichs bearbeitet werden, was besonders schonend ist.

Gemäß einer vorteilhaften Ausgestaltung kann das in den Zuführungsbereich eingeführte Arbeitsinstrument Bewegungsspiel zu dem den Zuführungsbereich begrenzenden Innenumfang der Arbeitskanüle im Sinne einer zum Innenumfang orthogonalen oder/und verkippenden Verstellung relativ zum Innenumfang aufweisen. Das Bewegungsspiel des Arbeitsinstruments ermöglicht eine Vergrößerung des Operationskanals an dessen durch das Fräsinstrument erzeugten Innenumfang, beispielsweise durch minimales Verkippen des Arbeitsinstruments bezogen auf die Achse der Längserstreckung der Arbeitskanüle, so dass der Arbeitsabschnitt des Arbeitsinstruments nicht um eine zentrale Achse der Arbeitskanüle gedreht wird. Dadurch lässt sich in der Regel auch Knochenmaterial bzw. Material des Bandscheibenvorfalls, das nach dem Fräsen am Innenumfang des Operationskanals vorhanden sein kann, vermittels des Arbeitsinstruments aus dem Operationskanal bzw. dem Operationsbereich entfernen.

Um die Operationstechnik zu verfeinern, wird vorgeschlagen, dass der Satz ferner wenigstens ein Zangeninstrument oder/und wenigstens ein Pinzetteninstrument aufweist, das durch das Einführende derart in den Zuführungsbereich einführbar ist, dass ein Zangenabschnitt bzw. Pinzettenabschnitt aus dem Austrittsende vorsteht und dass an wenigstens einem aus dem Einführende vorstehenden Betätigungsabschitt den Zangenabschnitt bzw. Pinzettenabschnitt betätigende Betätigungskräfte ausübbar sind. Das Zangeninstrument bzw. Pinzetteninstrument erlaubt die Entfernung von weiterem Material des Bandscheibenvorfalls, um den Bandscheibenvorfall beheben zu können. Die Ausführung des Zangeninstruments kann vorzugsweise derart sein, dass sowohl eine Schneidefunktion zum Wegnehmen von Material des Bandscheibenvorfalls, als auch eine Greiffunktion zum Festhalten und Herausziehen von Material des Bandscheibenvorfalls durch die Arbeitskanüle bereitgestellt wird. Ferner kann das Zangen- bzw. Pinzetteninstrument einen bezogen auf die Längserstreckung des Zangen- bzw. Pinzetteninstruments verstellbaren, wahlweise fixierbaren Anschlag aufweisen, der mit einem Anschlag der Arbeitskanüle an deren Einführende oder zu diesem benachbart zusammenwirkt, um eine gewählte maximale Länge des Vorstehens des Zangenabschnitts aus dem Austrittsende definieren zu können. Vorzugsweise kann der Anschlag auf einem Außenumfang des Zangen- bzw. Pinzetteninstruments angeordnet und an diesem vorzugsweise mittels einer Rändelschraube festgeklemmt werden.

Bevorzugterweise kann der Satz von Operationsinstrumenten ein der Arbeitskanüle zugeordnetes Abschlussinstrument aufweisen, welches durch das Einführende derart in den Zuführungsbereich einführbar und in einer Abschlussstellung positionierbar ist, dass aus dem Austrittsende ein ausgehend vom Austrittsende sich verjüngend ausgeführtes Abschlussende des Abschlussinstruments vorsteht und dass an einem aus dem Einführende vorstehenden Handhabungsende des Abschlussinstruments zumindest im Sinne eines Herausziehens des Abschlussinstruments aus der Arbeitskanüle wirkende Handhabungskräfte ausübbar sind. Das in der Abschlussstellung positionierte Abschlussinstrument ermöglicht das Verschließen der Arbeitskanüle, wobei das verjüngend ausgeführte Abschlussende eine Art (Einführ-)Spitze der Arbeitskanüle bildet, um diese leichter und ohne Gefahr von Verletzungen des Gewebes, von Nerven usw. in den Körper des Patienten in Richtung zum Operationsbereich einführen zu können.

In diesem Zusammenhang ist es zweckmäßig, wenn das Abschlussinstrument einen Außenumfang aufweist, der am Innenumfang der Arbeitskanüle linear führbar ist. Der dem Innenumfang der Arbeitskanüle angepasste Außenumfang des Abschlussinstruments erlaubt ein formschlüssiges Verschließen der Arbeitskanüle, insbesondere durch das vorstehende Abschlussende des Abschlussinstruments.

Ferner ist es bevorzugt, dass das Abschlussinstrument an der Arbeitskanüle gegen Verstellung aus der Abschlussstellung sicherbar ist. Die Sicherung gegen Verstellung aus der Abschlussstellung ermöglicht einen nicht veränderbaren Verschluss der Arbeitskanüle sowie die Bildung einer aus der Arbeitskanüle und dem Abschlussinstrument bestehenden, als Einheit handhabbaren Instrumenteneinheit.

Vorteilhafterweise ist zur Sicherung des Abschlussinstruments an der Arbeitskanüle eine Gewindeverbindung zwischen Abschlussinstrument und Arbeitskanüle vorgesehen, wobei vorzugsweise im Sinne einer Relativverdrehung zwischen Abschlussinstrument und Arbeitskanüle zum Herstellen bzw. Lösen der Gewindeverbindung wirkende Verdrehkräfte am Handhabungsende auf das Abschlussinstrument ausübbar sind. Durch die Gewindeverbindung können das Abschlussinstrument und die Arbeitskanüle bezogen auf die Längserstreckung der beiden Instrumente gegenseitig in einer bestimmten relativen Position zueinander festgehalten werden. Eine besonders vorteilhafte relative Position kann dadurch definiert sein, dass das Abschlussinstrument vollständig in die Arbeitskanüle eingeführt und die Gewindeverbindung bis zu einem Anschlag hergestellt ist.

Die Gewindeverbindung kann auf Grundlage eines Außengewindes des Abschlussinstruments am Handhabungsende oder zu diesem benachbart und auf Grundlage eines Innengewindes der Arbeitskanüle am Einführende oder zu diesem benachbart hergestellt werden. Durch die Verwendung des Außengewindes und des Innengewindes an den bevorzugten Positionen am Abschlussinstrument bzw. an der Arbeitskanüle kann bei vollständig festgezogener Gewindeverbindung die bereits oben erwähnte Instrumenteneinheit hergestellt werden. Diese Instrumenteneinheit weist ein Betätigungsende auf, das von den Betätigungsenden der Arbeitskanüle und des Abschlussinstruments gebildet sein kann.

Vorzugsweise können von dem in der Abschlussstellung positionierbar befindlichen Abschlussinstrument in Richtung zum Austrittsende gerichtete Vorschubkräfte auf die Arbeitskanüle ausgeübt werden. Durch die Ausübung von Vorschlubkräften kann die Arbeitskanüle zusammen mit dem Abschlussinstrument in den Operationsbereich eingeführt werden. Die durch das verjüngend ausgeführte Abschlussende des Abschlussinstruments gebildete konisch zulaufende Einführspitze dient in diesem Zusammenhang dazu, Verletzungen des Gewebes, von Gefäßen und Nerven beim Einführen der Arbeitskanüle in den Operationsbereich nach Möglichkeit zu verhindern, wie schon angesprochen.

In diesem Zusammenhang ist es vorteilhaft, wenn das Handhabungsende in der Abschlussstellung an dem Einführende formschlüssig anschlägt. Das formschlüssige Anschlagen des Handhabungsendes am Einführende in der Abschlussstellung ermöglicht eine direkte Übertragung von am Handhabungsende ausgeübten Vorschubkräften auf die Arbeitskanüle.

Das Handhabungsende und vorzugsweise das Einführende können mit einem massiven, ggf. flanschartigen Kopf ausgeführt sein. Eine solche Ausführung ermöglicht für das Ausüben von Vorschubkräften die Verwendung von entsprechenden Werkzeugen, beispielsweise von einem dafür geeigneten Hammer. Die Verwendung eines Hammers kann ggf. anstelle eines drehenden Hineindrückens der mit dem Abschlussinstrument verbundenen Arbeitskanüle bevorzugt sein.

Ferner ist es bevorzugt, dass das Abschlussinstrument rohrförmig ist. Die rohrförmige Ausbildung erhöht die mechanische Steifigkeit verglichen mit einer stabförmigen Ausführung, so dass entsprechende Vorschub- bzw. Torsionskräfte durch Beaufschlagen mit einem Hammer bzw. durch drehendes Hineindrücken optimal übertragen werden können, ohne dass das Abschlussinstrument durch die mechanische Beanspruchung verformt wird. Ferner ermöglicht die rohrförmige Ausbildung die Verwendung eines Führungsinstruments, wie im Folgenden noch näher erläutert.

Es ist besonders zweckmäßig, wenn der Arbeitskanüle ein an dieser bezogen auf ihre Längserstreckung verstellbarer, wahlweise an der Arbeitskanüle fixierbarer Anschlag zugeordnet ist, um eine gewählte maximale Länge des Einführens der Arbeitskanüle hin zu einem Operationsbereich zu definieren. Der verstellbare und wahlweise fixierbare Anschlag dient vorzugsweise als Tiefen-Anschlag und schlägt normalerweise außen auf der Haut eines Patienten an. Hierdurch kann erreicht werden, dass die Arbeitskanüle nicht zu tief eingebracht wird, was vor allem ein Schutz gegen Verletzung, insbesondere des Rückenmarks bzw. von Nervenwurzeln darstellt und was besonders schonend ist.

Vorteilhafterweise ist der Anschlag auf dem Außenumfang der Arbeitskanüle angeordnet und an dieser vorzugsweise mittels wenigstens einer Rändelschraube festklemmbar. Die Anordnung des verstellbaren Anschlags auf dem Außenumfang der Arbeitskanüle in Verbindung mit der zum Festklemmen vorgesehenen Rändelschraube führt zu einer einfachen Handhabung der Arbeitskanüle.

Der Satz kann vorteilhaft wenigstens ein der Arbeitskanüle oder/und dem in der Abschlussstellung angeordneten Abschlussinstrument zugeordnetes Führungsinstrument, ggf. Führungsrohr, umfassen. In diesem Zusammenhang ist es zweckmäßig, wenn das Führungsinstrument einen Außenumfang aufweist, an dem der Innenumfang der Arbeitskanüle bzw. des Abschlussinstruments linear führbar ist. Die Verwendung eines Führungsinstruments ermöglicht ein präzises, im Wesentlichen radialspielfreies Einführen der Arbeitskanüle bzw. des Abschlussinstruments, ggf. der oben erwähnten Instrumenteneinheit, in den Operationsbereich. Die lineare Führung durch das Führungsinstrument ist besonders vorteilhaft, um beim drehenden Hineindrücken oder beim Beaufschlagen mit dem Hammer der Arbeitskanüle bzw. des Abschlussinstruments ein durch Querkräfte verursachtes Verkippen aus der durch die Längserstreckung des Führungselements definierten Achse verhindern zu können.

Ferner kann der Satz ein dem Führungsrohr zugeordneten Führungsdraht aufweisen, über den das Führungsrohr schiebbar ist, um das Führungrohr in einer Führungsstellung zu positionieren. Es ist dabei besonders zweckmäßig, wenn ein Innenumfang des Führungsrohrs an einem Außenumfang des Führungsdrahts linear führbar ist. Vermittels des Führungsdrahts kann das Führungsrohr leichter eingeführt und zielgenau in einer Führungsstellung positioniert werden.

Bevorzugterweise weist der Satz auch eine Spinalnadel auf, in die der Führungsdraht schiebbar ist, um den Führungdraht in seiner Führungsstellung zu positionieren. Die Spinalnadel kann in der Regel leicht bis zum Bandscheibenvorfall bzw. bis zu einem wegzunehmenden Knochenabschnitt eingeführt werden. Durch die Spinalnadel wird eine erste, der Längserstreckung der Spinalnadel entsprechende Achse definiert. Der in die Spinalnadel eingeführte Führungsdraht nimmt dementsprechend seine durch diese Achse definierte Führungsstellung ein. Das über den Führungsdraht schiebbare Führungsrohr definiert und stabilisiert in seiner Führungsstellung die ursprünglich durch die Spinalnadel definierte Achse. Durch die bevorzugte lineare und im Wesentlichen radialspielfreie Führbarkeit des Führungsrohrs, des Abschlussinstruments, der Arbeitskanüle und der Fräsinstrumente wird ermöglicht, dass sich der Operationsbereich im Wesentlichen konzentrisch um die durch die Spinalnadel ursprünglich definierte Achse erreichen und bearbeiten lässt. Diese sukzessive konzentrische Vergrößerung des Zugangs zum Operationsbereich, bei der Körpergewebe verdrängt aber nicht durchtrennt wird, und dann die Bearbeitung des Operationsbereichs mittels der axial geführten Fräsinstrumente führt in der Regel zu einem schonenden, minimal operativen Eingriff für den Patienten. Die in der Arbeitskanüle im Wesentlichen radialspielfrei geführten Fräsinstrumente unterschiedlicher Länge ermöglichen dann die Verlängerung des Operationskanals bis zum Vorfall hin bzw. in diesen hinein, ohne dass dies zwingend eine Vergrößerung des Durchmessers des Operationskanals mit sich bringt. Zusätzlich kann allgemein durch die lineare Führbarkeit der oben erwähnten Instrumente die Handhabung des Satzes von Operationsinstrumenten einfach gehalten werden.

Die Spinalnadel kann vorzugsweise ein in diese einführbares, zum Schließen einer Öffnung einer Nadelspitze dienendes Trokar-Element aufweisen. Auf diese Weise kann verhindert werden, dass die Nadelöffnung Verletzungen verursacht, und dass Gewebe bzw. Material in das Innere der Nadel eintreten kann.

Nach einem anderen Aspekt betrifft die Erfindung eine Kombination aus mehreren Sätzen von Operationsinstrumenten, deren Instrumente unterschiedlichen Durchmesser oder/und unterschiedliche Länge aufweisen. Durch die Verwendung von Instrumenten verschiedener Größen und Längen kann für jeden Patient derjenige Instrumentensatz ausgewählt werden, mit dem einerseits der Bandscheibenvorfall optimal erreicht und entfernt werden kann und mit dem andererseits ein für den Patienten schonender, minimal operativer Eingriff ermöglicht wird.

Die Kombination zeichnet sich vorzugsweise dadurch aus, dass einander zugeordnete Instrumente eines jeweiligen Satzes den Satz identifizierende, optisch oder/und durch Tasten wahrnehmbare Kennzeichnungen aufweisen. Die Kennzeichnungen ermöglichen - wie dies bereits bei den Fräsinstrumenten erwähnt wurde - die rasche Identifikation eines einem bestimmten Satz von Operationsinstrumenten zugehörigen Instruments, wobei die Identifikation in der Regel einerseits durch den Operateur und andererseits durch dem Operateur beistehendes Assistenzpersonal erfolgt. Im Weiteren wird durch eine solche Kennzeichnung die Handhabung des Satzes von Operationsinstrumenten vereinfacht.

Es sei noch angemerkt, dass es für alle Instrumente des Instrumentensatzes bzw. für alle Instrumente der verschiedenen Sätze von Operationsinstrumenten generell vorteilhaft ist, wenn die Instrumente im Wesentlichen langgestreckt, geradlinig und steif bzw. starr ausgeführt sind.

Zum Durchführen von Wirbelsäulenoperationen, beispielsweise Operationen an der Halswirbelsäule, etwa zur Behebung von Bandscheibenvorfällen kann beispielsweise ein Satz von Operationsinstrumenten oder eine Kombination aus mehreren Sätzen von Operationsinstrumenten nach der Erfindung wie vorangehend beschrieben verwendet werden. Dabei kann vorteilhaft die oder eine vorzugsweise das Abschlussinstrument in der Abschlussstellung aufweisende Arbeitskanüle zu einem Operationsbereich eingeführt werden und anschließend kann, ggf. nach Entfernung des Abschlussinstruments, das bzw. ein Fräsinstrument durch die Arbeitskanüle hin zum Operationsbereich eingeführt werden. Es kann dann das Fräsinstrument durch Ausübung von Betätigungskräften auf das Betätigungsende betätigt werden. Das Einführen der das Abschlussinstrument in der Abschlussstellung aufweisenden Arbeitskanüle stellt eine besonders schonende Operationstechnik dar, insbesondere wenn das Abschlussinstrument die sich verjüngende Einführspitze aufweist. Die zum Operationsbereich hin eingeführte Arbeitskanüle kann jeweils ein Fräsinstrument radialspielfrei führbar aufnehmen, das idealerweise der jeweiligen Operationssituation angepasst ist.

Ferner kann bei einer solchen Operation die Arbeitskanüle unter Führung durch das oder ein in einer Führungstellung positioniertes Führungsinstrument, ggf. Führungsrohr, zum Operationsbereich zugeführt werden. Dabei ist es denkbar, dass das Führungsrohr unter Vermittlung eines gewünschtenfalls mittels einer Spinalnadel in einer Führungsstellung positionierten Führungdrahts in die Führungsstellung gebracht wird. Die Einführung des Führungsdrahts und des Führungsinstruments, die dann beide jeweils in ihrer Führungsstellung positioniert werden, kann zweckmäßigerweise als Vorbereitung für das Einführen des Abschlussinstruments und der Arbeitskanüle durchgeführt werden.

Weiter ist es denkbar, dass nacheinander mehrere Fräsinstrumente unterschiedlicher Länge durch die Arbeitskanüle hin zum Operationsbereich eingeführt und durch Ausübung von Betätigungskräften auf das jeweilige Betätigungsende betätigt werden. Durch die Verwendung von Fräsinstrumenten unterschiedlicher Länge kann der Operationsbereich entsprechend der Operationssituation schonend vergrößert werden.

Nach Betätigung wenigstens eines Fräsinstruments kann nach Entfernung desselben ein weiteres Arbeitsinstrument mit einem/dem löffelartigen oder/und schneidenartigen oder/und schaberartigen Arbeitsabschnitt durch die Arbeitskanüle hin zum Operationsbereich zugeführt werden und hiermit Material im Operationsbereich abgelöst oder/und ein Operationskanal vergrößert oder/und Material aus dem Operationsbereich entfernt werden. Das Arbeitsinstrument ermöglicht vorteilhafterweise auch die Vergrößerung des Operationskanals bzw. das Entfernen von Material aus dem Operationsbereich in Bereichen, welche durch das bzw. die Fräsinstrumente nicht bearbeitet werden können.

Ferner kann nach Betätigung wenigstens eines Fräsinstruments nach Entfernen desselben bzw. nach Entfernen des Arbeitsinstruments ein Zangeninstrument oder/und ein Pinzetteninstrument durch die Arbeitskanüle hin zum Operationsbereich zugeführt werden und hiermit Material im Operationsbereich abgelöst oder/und ein Operationskanal vergrößert oder/und Material aus dem Operationsbereich entfernt werden. Die Verwendung des Zangen- bzw. Pinzetteninstruments ermöglicht in der Regel die vollständige Behebung des Bandscheibenvorfalls und die vollständige Entfernung von durch die Operation angefallenem Knochenmaterial bzw. Material des Bandscheibenvorfalls.

Die Erfindung wird im Folgenden anhand eines in den Figuren 1 bis 8 gezeigten Ausführungsbeispiels näher erläutert. In den Figuren zeigt:
- Fig. 1: eine Spinalnadel mit einem nicht vollständig eingeführten Trokar-Element und einem Führungsdraht eines Satzes von Operationsinstrumenten;
- Fig. 2: ein als Führungsinstrument dienendes Führungsrohr und eine Arbeitskanüle mit mit zugehörigem Abschlussinstrument einzeln (Fig. 2a) und in Kombination (Fig. 2b) als weitere Bestandteile des Satzes von Operationsinstrumenten nach dem erfindungsgemäßen Ausführungsbeispiel;
- Fig. 3: in den Teilfiguren 3a, 3b und 3c drei Fräsinstrumente des Satzes von Operationsinstrumenten mit verschiedenen Längen, wobei das Fräsinstrument der mittleren Länge teilweise eingeführt in der Arbeitskanüle dargestellt ist (Fig. 3b);
- Fig. 4: eine Draufsicht auf das Betätigungselement eines Fräsinstruments entsprechend dem Pfeil IV in Fig. 3, wobei in den Teilfiguren 4a und 4b zwei mögliche Ausführungsvarianten dargestellt sind;
- Fig. 5: Ansichten der Betätigungselemente des Fräsinstruments nach zwei Ausführungsvarianten mit einer knebelartigen (Fig. 5a) bzw. knaufartigen (Fig. 5b) Ausgestaltung;
- Fig. 6: eine Querschnittsdarstellung in vergrößerter Form des Bereichs der Verbindung des Einführendes der Arbeitskanüle und des Betätigungselements des Abschlussinstruments gemäß Fig. 2b nach Schnittlinie VI-VI;
- Fig. 7: ein löffelartiges Arbeitsinstrument des Satzes von Operationsinstrumenten; und
- Fig.8: ein Zangeninstrument des Satzes von Operationsinstrumenten in verschiedenen Ansichten (Fig. 8a, b, c).

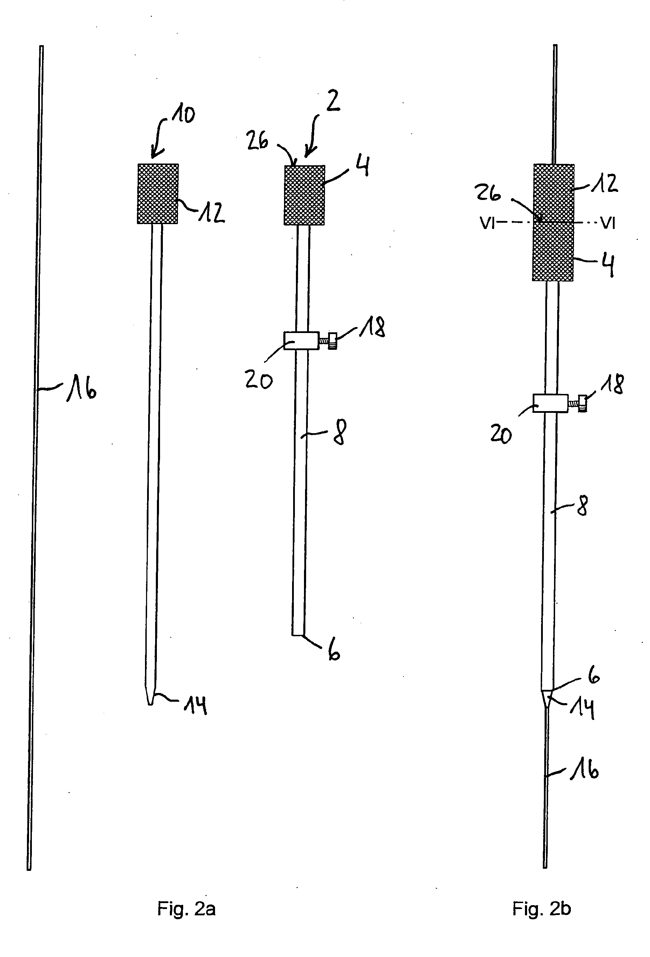

Eine in Fig. 2a dargestellte Arbeitskanüle 2 mit ihrem Einführende 4 und ihrem Austrittsende 6 sowie dem das Einführende 4 und das Austrittsende 6 verbindenden Zuführungsbereich 8 kann ein Abschlussinstrument 10 linear führbar aufnehmen. Das Abschlusselement 10 weist ein Handhabungsende 12 und ein sich verjüngend ausgeführtes Abschlussende 14 auf. Das Abschlussinstrument 10 dient dazu, den Zwischenraum zwischen einem rohrartigen Führungsinstrument 16 und der Arbeitskanüle 2 am Austrittsende 6 zu verschließen, unter Ausbildung einer vom Durchmesser der Arbeitskanüle 2 zum Durchmesser des im Folgenden auch als Führungsrohr bezeichneten Führungsinstruments 16 konisch zulaufenden Einführspitze 14 des Abschlussinstruments. Diese Ausbildung dient dazu, Verletzungen des Gewebes, von Gefäßen und Nerven beim Einführen der Arbeitskanüle 2 zu verhindern. Ohne das Abschlussinstrument 10 hätte die Arbeitskanüle 2 ein stumpfes oder sogar schneidenartiges Ende im Sinne einer Ringschneide.

Die Arbeitskanüle 2 trägt auf ihrem Außenumfang einen in Längsrichtung verstellbaren, mittels einer Rändelschraube 18 in einer gewählten Längsposition fixierbaren Anschlagring 20. Dieser dient als Tiefen-Anschlag, und schlägt außen auf der Haut des Patienten an. Es wird hierdurch erreicht, dass die Arbeitskanüle 2 nicht zu tief eingebracht wird (Schutz gegen Verletzung, insbesondere des Rückenmarks bzw. der Nervenwurzel).

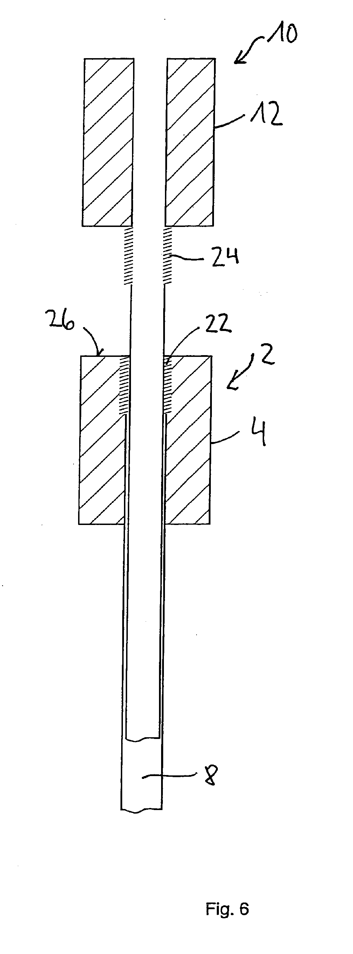

Damit das Abschlussinstrument 10 beim Heranführen der Arbeitskanüle 2 an den Operationsbereich, beispieslweise Bandscheibenvorfall, nicht vom Austrittsende 6 der Arbeitskanüle 2 nach innen weggedrückt wird, ist eine Gewindeverbindung zwischen dem Abschlussinstrument 10 und der Arbeitskanüle 2 vorgesehen, mit einem in Fig. 6 dargestellten Innengewinde 22 im Einführende 12 der Arbeitskanüle 2 und einem Außengewinde 24 auf dem Außenumfang des Abschlussinstruments 10 knapp unterhalb dessen Handhabungsende 12.

In Fig. 2b ist das Abschlussinstrument 10 in den Zuführungsbereich 8 der Arbeitskanüle 2 eingeschoben, wobei durch das in Fig. 6 dargestellte Innengewinde 22 und das Außengewinde 24 eine Gewindeverbindung zwischen der Arbeitskanüle 2 und dem Abschlussinstrument 10 hergestellt ist. Die vollständig angezogene Gewindeverbindung definiert eine Abschlussstellung des Handhabungsendes 12 des Abschlussinstruments 10 an einem ortsfesten Anschlag 26 des Einführendes 4 der Arbeitskanüle 2. Durch die Arbeitskanüle 2 und das Abschlussinstrument 10 ist auf diese Weise eine Instrumenteneinheit gebildet, die über das Führungsinstrument 16 geschoben werden kann, wie in Fig. 2b gezeigt. Nach Positionierung der Instrumenteneinheit in Zuordnung zu einem Operationsbereich wird das Abschlussinstrument 10 entfernt, um den Zugang zum Operationsbereich durch die Arbeitskanüle 2 hindurch für weitere Operationsinstrumente freizugeben.

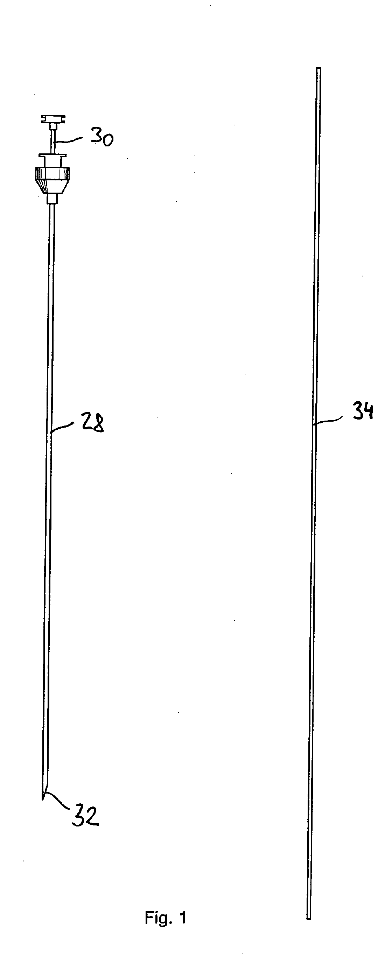

Um das Führungsinstrument 16 und folglich auch die das Abschlussinstrument 10 in seiner Abschlussstellung enthaltende Arbeitskanüle 2 dem Operationsbereich zuführen zu können, kann vorteilhaft eine Spinalnadel 28, die ein Trokar-Element 30 enthält, wie dies in Fig. 1 dargestellt ist, dem Operationsbereich zugeführt werden. Das Trokar-Element 30 verschließt die Öffnung an der Nadelspitze 32, so dass die Nadelöffnung 32 keine Verletzungen verursachen kann und kein Material in das Innere der Nadel 28 eintritt. Das Trokar-Element 30 wird nach Einführen der Spinalnadel 28 durch einen Führungsdraht 34 ersetzt, über den das rohrartige Führungsinstrument 16 linear führbar geschoben wird.

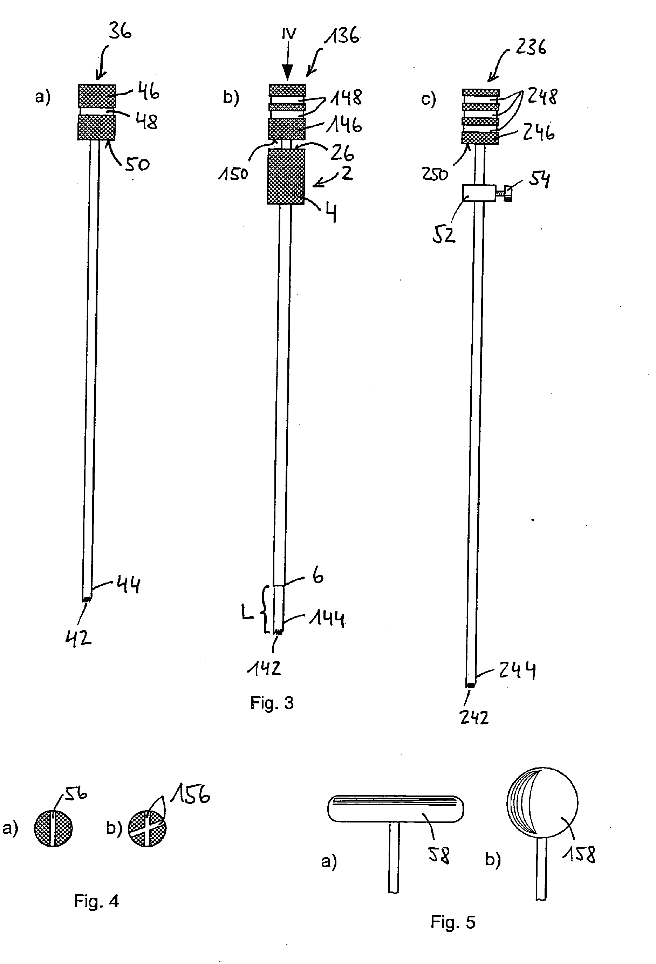

Fig. 3 zeigt in Fig. 3a ein Fräsinstrument 36 und entsprechende Ausführungsvarianten unterschiedlicher Länge, bezeichnet mit 136 bzw. 236, in den in Fig. 3b und 3c. Alle Fräsinstrumente 36, 136, 236 weisen einen ringsägeartigen Fräsabschnitt 42, 142, 242 am Fräsende 44, 144, 244 auf. Ferner zeigen alle Fräsinstrumente 36, 136, 236 entsprechende Betätigungsenden 46, 146, 246 auf, die durch das jeweilige Fräsinstrument identifizierende, optisch oder/und durch Tasten wahrnehmbare Kennzeichnungen 48, 148, 248 in Form einer oder mehrerer Ringnuten unterschieden werden können. Das Fräsinstrument 136 ist in Fig. 3b in teilweise eingeschobenem Zustand in der Arbeitskanüle 2 dargestellt, wobei das Fräsende 144 um eine Länge L aus dem Austrittsende 6 der Arbeitskanüle 2 vorsteht. Jedes der Fräsinstrumente 36, 136, 236 weist auf der Unterseite des jeweiligen Betätigungsendes 46, 146, 246 einen ortsfesten Anschlag 50, 150, 250 auf, der auf dem Anschlag 26 aufliegen kann und damit eine maximale wohldefinierte Länge Lₘₐₓ des Vorstehens des Fräsendes 44, 144, 244 aus dem Austrittsende 60 festlegt. Das Fräsinstrument 236 in Fig. 3c weist einen verstellbaren, wahlweise fixierbaren Anschlag 52 auf, mit einer zur Fixierung des Anschlags 52 vorgesehenen Rändelschraube 54 zur Festlegung einer wählbaren maximalen Länge L_{gmax} des Vorstehens.

Fig. 4a, 4b zeigt eine Draufsicht entsprechend dem Pfeil IV in Fig. 3, in der zwei Ausführungsvarianten der einen jeweiligen Satz von Operationsinstrumenten identifizierenden, optisch oder/und durch Tasten wahrnehmbaren Kennzeichnung 56, 156 erkennbar sind. Die Kennzeichnung 56, 156 kann in entsprechender Form an jedem der zu einem Satz von Operationsinstrumenten gehörenden Instrumente vorgesehen sein zur vereinfachten Identifizierung und Handhabung eines Satzes von Operationsinstrumenten.

Zwei mögliche Ausführungsvarianten des Betätigungsendes 46, 146, 246 für die Fräsinstrumente 36, 136, 236 sind in Fig. 5 dargestellt, wobei Fig. 5a ein knebelartiges Betätigungsende 58 und Fig. 5b ein knaufartiges Betätigungsende 158 zeigt, die eine einfachere Ausübung von drehenden Betätigungskräften ermöglichen.

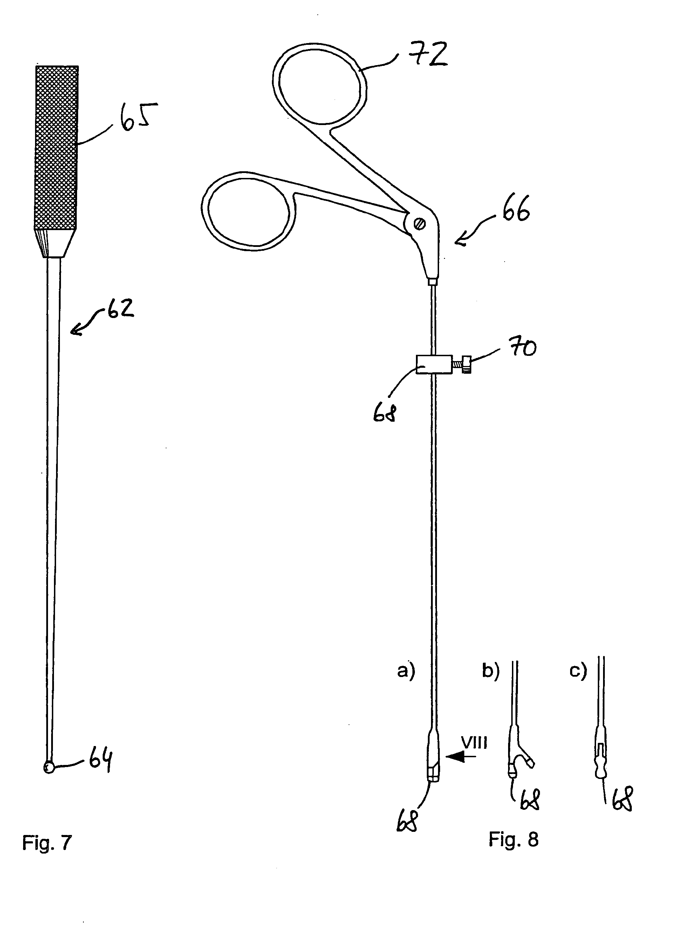

Ein in Fig. 7 dargestelltes Arbeitsinstrument 62 mit einem löffelartigen Arbeitsabschnitt 64 und einem Betätigungsende 65 kann in die Arbeitskanüle 2 eingeführt werden, um den Operationsbereich vergrößern bzw. aus diesem Material entfernen zu können.

Fig. 8a zeigt ein Zangen- bzw. Pinzetteninstrument 66 mit seinem Zangen- bzw. Pinzettenabschnitt 68, wobei der Zangen- bzw. Pinzettenabschnitt in Fig. 8b auch in seiner geöffneten Position dargestellt ist. Fig. 8c zeigt eine Draufsicht auf den Zangen- bzw. Pinzettenabschnitt entsprechend dem Pfeil VIII. Das Zangeninstrument 66 kann ebenfalls einen verstellbaren, wahlweise fixierbaren Anschlag 68 aufweisen, der mittels einer Rändelschraube 70 festgeklemmt werden kann. Das Öffnen des Zangen- bzw. Pinzettenabschnitts erfolgt durch Betätigung eines scherenartigen Betätigungsabschnitts 72. Das Zangen- bzw. Pinzetteninstrument 66 ermöglicht unter anderem eine gezielte Entnahme von Material aus dem Operationsbereich durch die Arbeitskanüle 2.

Die in den Figuren 1 bis 8 aufgeführten Instrumente weisen insbesondere für eine Halswirbelsäulenoperation beispielsweise vorteilhaft folgende Dimensionen auf für die ineinander schiebbaren Abschnitte ohne Betätigungs-/Handhabungsenden:

| | ***Länge*** | ***Innendurchmesser*** | ***Außendurchmesser*** |
|---|---|---|---|
| Arbeitskanüle | 85 - 105 mm | 2,0 - 4,5 mm | 2,5 - 5,0 mm |
| Abschlussinstrument | 90 - 110 mm | 1,5 - 4,0 mm | 2,0 - 4,5 mm |
| Fräsinstrument | 100 - 125 mm | 1,5 - 4,0 mm | 2,0 - 4,5 mm |
| Arbeitsinstrument | 110 - 220 mm | 1,5 - 4,0 mm (Löffeldurchmesser) | 2,0 - 4,5 mm (Löffendurchmesser) |
| Zangeninstrument | 140 - 280 mm | | |
| Führungsinstrument | 200 - 270 mm | 0,5 - 1,5 mm | 1,0 - 2,0 mm |
| Führungsdraht | 250 - 320 mm | | 0,5 - 1,0 mm |
| Spinalnadel | 100 - 150 mm | 0,5 - 1,0 mm | 1,0 - 1,5 mm |

Bevorzugt werden mehrere Sätze von Operationsinstrumenten unterschiedlicher Dimensionierung bereitgestellt, vorzugsweise mit gestaffelten Dimensionierungen innerhalb der in der vorstehenden Tabelle angegebenen Bemessungsbereichen.

Eine bevorzugte Vorgehensweise bei einer Operation an der Wirbelsäule, insbesondere Halswirbelsäule, ist wie folgt:

Als erstes wird die Spinalnadel 28, auch Hohlnadel genannt, mit dem darin enthaltenen Trokar-Element 30 bis zum Bandscheibenvorfall bzw. bis zum wegzunehmenden Knochenabschnitt eingeführt.

Nach Platzieren der Spinalnadel 28 wird das Trokar-Element 30 herausgezogen. Es können nun, soweit angezeigt, Medikamente oder/und Kontrastmittel durch die Spinalnadel dem Operationsbereich zugeführt werden.

In die Spinalnadel 28 wird der Führungsdraht 34 eingeführt, bis zur Spitze 32 der Spinalnadel 28 bzw. ggf. bis in den Operationsbereich, ggf. Vorfallsbereich, hinein.

Über den Führungsdraht 34 wird ein dünnes Führungsrohr bzw. Führungsinstrument 16 geschoben, bis zum Knochen, der weggenommen werden muss, bzw. bis zum Operationsbereich (Vorfall). Der Führungsdraht 34 kann verbleiben oder entfernt werden.

Über das Führungsrohr 16 wird dann die ineinander eingeführte Instrumenteneinheit 2, 10 geschoben. Die Instrumenteneinheit ist gebildet von der außen liegenden Arbeitskanüle 2 und dem darin eingeführten, als "Spacer-Röhrchen" bezeichenbaren Abschlussinstrument 10. Beim Einführen der Instrumenteneinheit in den Operationsbereich kann es vorteilhaft sein, anstelle eines drehenden Hineindrückens die Instrumenteneinheit an ihrem äußeren Ende (Handhabungsende 12) mit einem Hammer zu beaufschlagen, wofür das Handhabungsende 12 und das Einführende 4 mit einem stabilen Flansch ausgeführt sind.

Nach Platzieren der Arbeitskanüle 18 einschließlich des Abschlussinstruments 10 wird dieses und - zumindest in der Regel - das Führungsrohr 16 herausgezogen. Durch die Arbeitskanüle 2 können dann nacheinander verschiedene Fräsinstrumente des Satzes von Operationsinstrumenten, etwa die Fräsen 36, 136, 236 gemäß Fig. 3, eingeführt werden, um Knochenmaterial oder/und Material des Bandscheibenvorfalls ("Hernia") wegzufräsen. Als erstes kommt bevorzugt die kurze Fräse 36 ("1er-Fräse", beispielsweise gekennzeichnet durch eine Ringnut 48 im Betätigungsende 46) zum Einsatz, die bei maximalem Einschub in die Kanüle 2 genauso oder sogar weniger aus der Kanüle 2 vorsteht als das entfernte Abschlussinstrument 10, wodurch eine hohe Sicherheit erreicht wird. Die kleinste Fräse 36 weist einen dem Innendurchmesser der Arbeitskanüle 2 entsprechenden Außendurchmesser auf.

Als nächstes kann die längere Fräse 136 ("2er-Fräse") eingeführt werden, die bei maximalem Einschub um eine Länge Lₘₐₓ aus der Arbeitskanüle 2 vorsteht. Lₘₐₓ ist im Hinblick auf eine typische Bandscheibenvorfallsituation gerade so bemessen, dass die Fräse 136 tief in den Vorfall hineinreichen kann. Diese Bemessung geht davon aus, dass beim Einführen der Kanüle 2 einschließlich des Abschlussinstruments 10 die Spitze 14 des Abschlussinstruments bis an oder in den Vorfall hinein eingeführt wurde, und dass die Arbeitskanüle 12 dann knapp vor dem Vorfall endete.

Für manche Situationen kann auch die längere Fräse 236 ("3er-Fräse") zum Einsatz kommen, die deutlich weiter aus der Arbeitskanüle 2 vorstehen kann und insbesondere die gesamte Breite einer Bandscheibe überbrücken kann. Eine solche Fräse 236 wird verwendet, wenn man in manchen Situationen mit der Arbeitskanüle 2 nicht bis zum Bandscheibenvorfall vorstoßen kann. Die lange Fräse 236 ist mit einem durch eine Rändelschraube 54 feststellbaren Anschlagring 52 versehen, der am Einführende 4 der Arbeitskanüle 2 anschlägt und als Tiefenanschlag dient.

Alle Fräsen weisen den gleichen Außenumfang auf und können im Wesentlichen radialspielfrei in die Arbeitskanüle eingeführt werden.

Nach den Fräsarbeiten kann durch die Arbeitskanüle 2 vorteilhaft das beispielsweise als scharfer Löffel ausgeführte Arbeitsinstrument 62 eingeführt und darin gedreht werden, um aus dem gefrästen Kanal darin enthaltenes Material wegzunehmen bzw. den Kanal zu vergrößern.

Anschließend kann mittels eines durch die Arbeitskanüle eingeführten Zangen- bzw. Pinzetteninstruments 66 weiteres Material entfernt werden, etwa um den Bandscheibenvorfall zu beheben.

Die Reihenfolge der Vorgehensschritte kann ggf. den Operationsbedürfnissen angepasst und verändert werden, so dass beispielsweise nach dem Einsatz der Fräsinstrumente (36, 136, 236) direkt das Zangen- bzw. Pinzetteninstrument 66 verwendet werden kann. Es brauchen bei einer Operation nicht alle angesprochenen Instrumente zum Einsatz kommen.

Nach der Erfindung wird unter anderem bereitgestellt ein Satz von Operationsinstrumenten zur Verwendung bei Wirbelsäulenoperationen, insbesondere Halswirbelsäulenoperationen, der eine Arbeitskanüle und wenigstens ein Fräsinstrument umfasst. Das Fräsinstrument ist derart in ein Einführende der Arbeitskanüle einführbar, dass ein Fräsende aus einem Austrittsende der Arbeitskanüle vorsteht und dass an einem Betätigungsende des Fräsinstruments Betätigungskräfte auf das Fräsinstrument ausübbar sind.

## Patentansprüche

1. Satz von Operationsinstrumenten für Wirbelsäulenoperationen, umfassend:
eine Arbeitskanüle (2), die ein Einführende (4) und Austrittsende (6) und einen das Einführende (4) und das Austrittsende (6) verbindenden inneren Zuführungsbereich (8) aufweist,
mehrere Fräsinstrumente (36, 136, 236) unterschiedlicher Länge, jeweils mit einem Fräsende (44; 144; 244) und einem Betätigungsende (46; 146; 246), wobei die Fräsinstrumente (36, 136, 236) jeweils einen Außenumfang aufweisen, der an einem Innenumfang der Arbeitskanüle (2) linear führbar ist, wobei die Fräsinstrumente (36, 136, 236) jeweils durch das Einführende (4) derart in den Zuführungsbereich (8) einführbar sind, dass das jeweilige Fräsende (44; 144; 244) aus dem Austrittsende (6) vorsteht, wobei die Fräsinstrumente (36, 136, 236)unterschiedlicher Länge bei maximaler Einführung in die Arbeitskanüle (2) mit ihrem Fräsende (44; 144; 244) unterschiedlich weit aus dem Austrittsende (6) vorstehen, und dass an dem jeweiligen aus dem Einführende (4) vorstehenden Betätigungsende (46; 146; 246) im Sinne einer Drehbewegung relativ zur Arbeitskanüle (2) oder/und im Sinne einer Verschiebebewegung relativ zur Arbeitskanüle (2) wirkende Betätigungskräfte auf das jeweilige Fräsinstrument (36; 136; 236) ausübbar sind.

2. Satz von Operationsinstrumenten für Wirbelsäulenoperationen, umfassend:
eine Arbeitskanüle (2), die ein Einführende (4) und Austrittsende (6) und einen das Einführende (4) und das Austrittsende (6) verbindenden inneren Zuführungsbereich (8) aufweist,
wenigstens ein Fräsinstrument (36; 136; 236) mit einem Fräsende (44; 144; 244) und einem Betätigungsende (46; 146; 246),
wobei das Fräsinstrument (36; 136; 236) durch das Einführende (4) derart in den Zuführungsbereich (8) einführbar ist, dass das Fräsende (44; 144; 244) aus dem Austrittsende (6) vorsteht und dass an dem aus dem Einführende (4) vorstehenden Betätigungsende (46; 146; 246) im Sinne einer Drehbewegung relativ zur Arbeitskanüle (2) oder/und im Sinne einer Verschiebebewegung relativ zur Arbeitskanüle (2) wirkende Betätigungskräfte auf das Fräsinstrument (36; 136; 236) ausübbar sind.

3. Satz nach Anspruch 2, **dadurch gekennzeichnet, dass** das Fräsinstrument (36; 136; 236) einen Außenumfang aufweist, der an einem Innenumfang der Arbeitskanüle (2) linear führbar ist.

4. Satz nach Anspruch 2 oder 3, **dadurch gekennzeichnet, dass** der Satz mehrere Fräsinstrumente (36; 136; 236) unterschiedlicher Länge umfasst.

5. Satz nach Anspruch 1 oder 4, **dadurch gekennzeichnet, dass** die Fräsinstrumente (36; 136; 236) unterschiedlicher Länge das jeweilige Fräsinstrument (36; 136, 236) identifizierende, optisch oder/und durch Tasten wahrnehmbare Kennzeichnungen (48; 148; 248) aufweisen.

6. Satz nach einem der Ansprüche 1 bis 5, **dadurch gekennzeichnet, dass** das Fräsinstrument (36; 136; 236) oder wenigstens eines der Fräsinstrumente (36; 136; 236) am Betätigungsende (46; 146; 246) oder zu diesem benachbart einen bezogen auf die Längserstreckung des Fräsinstruments (36; 136; 236) ortsfesten Anschlag (50; 150; 250) aufweist, der mit einem Anschlag (26) der Arbeitskanüle (2) an deren Einführende (4) oder zu diesem benachbart zusammenwirkt, um eine maximale Länge (Lₘₐₓ) des Vorstehens des Fräsendes (44; 144; 244) aus dem Austrittsende (6) zu definieren.

7. Satz nach einem der Ansprüche 1 bis 6, **dadurch gekennzeichnet, dass** das Fräsinstrument (36; 136; 236) oder wenigstens eines der Fräsinstrumente (36; 136; 236) am Betätigungsende (46; 146; 246) oder zu diesem benachbart einen bezogen auf die Längserstreckung des Fräsinstruments (36; 136; 236) verstellbaren, wahlweise fixierbaren Anschlag (52) aufweist, der mit einem Anschlag (26) der Arbeitskanüle (2) an deren Einführende (4) oder zu diesem benachbart zusammenwirkt, um eine gewählte maximale Länge (L_{gmax}) des Vorstehens des Fräsendes (44; 144; 244) aus dem Austrittsende (6) zu definieren.

8. Satz nach Anspruch 7, **dadurch gekennzeichnet, dass** der Anschlag (52) auf dem Außenumfang des Fräsinstruments (36; 136; 236) angeordnet und an diesem vorzugsweise mittels wenigstens einer Rändelschraube (54) festklemmbar ist.

9. Satz nach einem der Ansprüche 1 bis 8, **dadurch gekennzeichnet, dass** das Fräsinstrument (36; 136; 236) oder wenigstens eines der Fräsinstrumente (36; 136; 236) als Hohlinstrument mit einem ringsägeartigen Fräsabschnitt (42; 142; 242) am Fräsende (44; 144; 244) ausgeführt ist.

10. Satz nach einem der Ansprüche 1 bis 9, **dadurch gekennzeichnet, dass** das Fräsinstrument (36; 136; 236) oder wenigstens eines der Fräsinstrumente (36; 136, 236) ein knauf- oder knebelartiges Betätigungsende (58; 158) aufweist.

11. Satz nach einem der Ansprüche 1 bis 10, **dadurch gekennzeichnet, dass** der Satz ferner wenigstens ein Arbeitsinstrument (62) mit einem löffelartigen oder/und schneidenartigen oder/und schaberartigen Arbeitsabschnitt (64) umfasst, welches durch das Einführende (4) derart in den Zuführungsbereich (8) einführbar ist, dass der Arbeitsabschnitt (64) aus dem Austrittsende (6) vorsteht und dass an einem aus dem Einführende (4) vorstehenden Betätigungsende (65) im Sinne einer Drehbewegung relativ zur Arbeitskanüle (2) oder/und im Sinne einer Verschiebebewegung relativ zur Arbeitskanüte (2) wirkende Betätigungskräfte auf das Arbeitsinstrument (62) ausübbar sind.

12. Satz nach Anspruch 11, **dadurch gekennzeichnet, dass** das in den Zuführungsbereich (8) eingeführte Arbeitsinstrument (62) Bewegungsspiel zu dem den Zuführungsbereich (8) begrenzenden Innenumfang der Arbeitskanüle (2) im Sinne einer zum Innenumfang orthogonalen oder/und verkippenden Verstellung relativ zum Innenumfang aufweist.

13. Satz nach einem der Ansprüche 1 bis 12, **dadurch gekennzeichnet, dass** der Satz ferner wenigstens ein Zangeninstrument oder/und wenigstens ein Pinzetteninstrument (66) aufweist, das durch das Einführende (4) derart in den Zuführungsbereich (8) einführbar ist, dass ein Zangenabschnitt bzw. Pinzettenabschnitt (68) aus dem Austrittsende (6) vorsteht und dass an wenigstens einem aus dem Einführende (4) vorstehenden Betätigungsabschitt (72) den Zangenabschnitt bzw. Pinzettenabschnitt (68) betätigende Betätigungskräfte ausübbar sind.

14. Satz nach einem der Ansprüche 1 bis 13, **dadurch gekennzeichnet, dass** der Satz ein der Arbeitskanüle (2) zugeordnetes Abschlussinstrument (10) aufweist, welches durch das Einführende (4) derart in den Zuführungsbereich (8) einführbar und in einer Abschlussstellung positionierbar ist, dass aus dem Austrittsende (6) ein ausgehend vom Austrittsende (6) sich verjüngend ausgeführtes Abschlussende (14) des Abschlussinstruments (10) vorsteht und dass an einem aus dem Einführende (4) vorstehenden Handhabungsende (12) des Abschlussinstruments (10) zumindest im Sinne eines Herausziehens des Abschlussinstruments (10) aus der Arbeitskanüle (2) wirkende Handhabungskräfte ausübbar sind.

15. Satz nach Anspruch 14, **dadurch gekennzeichnet, dass** das Abschlussinstrument (10) einen Außenumfang aufweist, der am Innenumfang der Arbeitskanüle (2) linear führbar ist.

16. Satz nach Anspruch 14 oder 15, **dadurch gekennzeichnet, dass** das Abschlussinstrument (10) an der Arbeitskanüle (2) gegen Verstellung aus der Abschlussstellung sicherbar ist.

17. Satz nach Anspruch 16, **dadurch gekennzeichnet, dass** zur Sicherung des Abschlussinstruments (10) an der Arbeitskanüle (2) eine Gewindeverbindung zwischen Abschlussinstrument (10) und Arbeitskanüle (2) vorgesehen ist, wobei vorzugsweise im Sinne einer Relativverdrehung zwischen Abschlussinstrument (10) und Arbeitskanüle (2) zum Herstellen bzw. Lösen der Gewindeverbindung wirkende Verdrehkräfte am Handhabungsende (12) auf das Abschlussinstrument (10) ausübbar sind.

18. Satz nach Anspruch 16 oder 17, **dadurch gekennzeichnet, dass** die Gewindeverbindung auf Grundlage eines Außengewindes (24) des Abschlussinstruments (10) am Handhabungsende (12) oder zu diesem benachbart und auf Grundlage eines Innengewindes (22) der Arbeitskanüle (2) am Einführende (4) oder zu diesem benachbart herstellbar ist.

19. Satz nach einem der Ansprüche 14 bis 18, **dadurch gekennzeichnet, dass** von dem in der Abschlussstellung positionierbar befindlichen Abschlussinstrument (10) in Richtung zum Austrittsende (6) gerichtete Vorschubkräfte auf die Arbeitskanüle (2) ausübbar sind.

20. Satz nach Anspruch 19, **dadurch gekennzeichnet, dass** das Handhabungsende (12) in der Abschlussstellung an dem Einführende (4) formschlüssig anschlägt.

21. Satz nach Anspruch 20, **dadurch gekennzeichnet, dass** das Handhabungsende (12) und vorzugsweise das Einführende (4) mit einem massiven, ggf. flanschartigen Kopf ausgeführt ist.

22. Satz nach einem der Ansprüche 14 bis 21, **dadurch gekennzeichnet, dass** das Abschlussinstrument (10) rohrförmig ist.

23. Satz nach einem der Ansprüche 1 bis 22, **dadurch gekennzeichnet, dass** der Arbeitskanüle (2) ein an dieser bezogen auf ihre Längserstreckung verstellbarer, wahlweise an der Arbeitskanüle (2) fixierbarer Anschlag (20) zugeordnet ist, um eine gewählte maximale Länge des Einführens der Arbeitskanüle (2) hin zu einem Operationsbereich zu definieren.

24. Satz nach Anspruch 23, **dadurch gekennzeichnet, dass** der Anschlag (20) auf dem Außenumfang der Arbeitskanüle (2) angeordnet und an dieser vorzugsweise mittels wenigstens einer Rändelschraube (18) festklemmbar ist.

25. Satz nach einem der Ansprüche 1 bis 24, **gekennzeichnet durch** wenigstens ein der Arbeitskanüle (2) oder/und dem in der Abschlussstellung angeordneten Abschlussinstrument (10) zugeordnetes Führungsinstrument (16), ggf. Führungsrohr.

26. Satz nach Anspruch 25, **dadurch gekennzeichnet, dass** das Führungsinstrument (16) einen Außenumfang aufweist, an dem der Innenumfang der Arbeitskanüle (2) bzw. des Abschlussinstruments (10) linear führbar ist.

27. Satz nach Anspruch 25 oder 26, **gekennzeichnet durch** ein dem Führungsrohr (16) zugeordneter Führungsdraht (34), über den das Führungsrohr (16) schiebbar ist, um das Führungrohr (16) in einer Führungsstellung zu positionieren.

28. Satz nach Anspruch 27, **dadurch gekennzeichnet, dass** ein Innenumfang des Führungsrohrs (16) an einem Außenumfang des Führungsdrahts (34) linear führbar ist.

29. Satz nach Anspruch 27 oder 28, **gekennzeichnet durch** eine Spinalnadel (28), in die der Führungsdraht (34) schiebbar ist, um den Führungdraht (34) in einer Führungsstellung zu positionieren.

30. Satz nach Anspruch 29, **gekennzeichnet durch** ein in die Spinalnadel (28) einführbares, zum Schließen einer Öffnung einer Nadelspitze (32) dienendes Trokar-Element (30).

31. Satz nach einem der vorhergehenden Ansprüche, vorgesehen für Operationen an der Halswirbelsäule.

32. Kombination aus mehreren Sätzen von Operationsinstrumenten nach einem oder mehreren der vorhergehenden Ansprüchen, deren Instrumente unterschiedlichen Durchmesser oder/und unterschiedliche Länge aufweisen.

33. Kombination nach Anspruch 32, **dadurch gekennzeichnet, dass** einander zugeordnete Instrumente eines jeweiligen Satzes den Satz identifizierende, optisch oder/und durch Tasten wahrnehmbare Kennzeichnungen (56; 156) aufweisen.
